# EUROPEAN PATENT APPLICATION

(11) **EP 1 621 550 A1**
(43) Date of publication of application: **01.02.2006**
(21) Application number: 04017907.9
(22) Date of filing: 29.07.2004
(51) Int. Cl.: C07K 14/705, C12N 5/06

(54) **Tumor-homing cells engineered to produce tumor necrosis factor-related apoptosis-inducing ligand (TRAIL)**

(71) Applicant: Dompé S.P.A., I-67100 L'Aquila (IT)
(72) Inventor: Gianni, Alessandro M., 20133 Milano (IT); Carlo-Stella, Carmelo, 20133 Milano (IT); Colotta, Francesco, 67100 L'Aquila (IT)
(74) Representative: Minoja, Fabrizio

(57) **Abstract**

The invention refers to tumor-homing cells expressing the tumor necrosis factor-related apoptosis inducing ligand (TRIAL), particularly to CD34+ and NK cells transduced with adenoviral vectors coding for TRIAL. The cells of the invention are useful for the intravenous treatment of tumors, particularly of lymphoma.

## Description

### FIELD OF THE INVENTION

The present invention relates to tumor-homing cells expressing the tumor necrosis factor-related apoptosis inducing ligand (TRAIL) and the use thereof in anti-tumor therapy.

### BACKGROUND OF THE INVENTION

Dysregulated apoptosis mechanisms play key roles in the pathogenesis and progression of lymphoproliferative disorders, allowing neoplastic cells to survive beyond their normal life-span, and to eventually acquire chemo-radioresistance [1]. Thus, apoptotic pathways represent attractive therapeutic targets for restoring apoptosis sensitivity of malignant cells or activating agonists of apoptosis [2]. In order to modulate apoptotic genes and proteins, several strategies can be envisaged which target either the intrinsic (Bcl-2, Bcl-X_{L}), extrinsic (DR4, DR5, FLIP), or the convergence (cIAP2) pathways of apoptosis [3].

Tumor necrosis factor-related apoptosis-inducing ligand (TRAIL) belongs to the TNF family of death receptor ligands [4].

Isolated DNA sequences coding for TRAIL, expression vectors comprising said DNA sequences and recombinant TRAIL polypeptides are disclosed in WO 97/01633.

TRAIL binds to death receptor 4 (DR4) and death receptor 5 (DR5), which are expressed on the cell surface of many cancer cells [5]. Binding soluble TRAIL to DR4 or DR5 leads to to caspase activation and apoptosis [6]. In vitro and in vivo studies suggest that due to a high cancer cell-specificity as well as a potent antitumor activity, soluble recombinant TRAIL might play a key role in anti-cancer therapy [7]. In fact, TRAIL selectively induces apoptosis in many transformed cells, while sparing normal cells [4]. Additionally, TRAIL administration in mice exerts a remarkable efficacy against tumor xenografts of colon carcinoma [8, 9], breast cancer [10], multiple myeloma [11], or glioma [12, 13].

Toxicity studies in mice and nonhuman primates have demonstrated that normal hepatocytes and keratinocytes are resistant to trimerized version of TRAIL [14, 15], whereas they show significant sensitivity to apoptosis induction by non-optimized TRAIL preparations or antibody-crosslinked variants of the ligand [16]. Whether or not hepatic toxicity could represent a major problem when using a trimerized version of TRAIL at high doses remains controversial.

An additional limitation to the use of soluble TRAIL is represented by the impaired apoptotic response observed in a substantial number and variety of tumor cell lines which require either a prolonged incubation time or very high dose of soluble TRAIL to undergo apoptosis [17, 18]. Given the pharmacokinetic profile of TRAIL after intravenous injection (plasmatic half-life of 32 minutes and elimination half-life of 4.8 hours), conditions of prolonged exposure time and high concentrations are not transferable for any in vivo treatment strategy [10]. In order to overcome limitations related to soluble recombinant TRAIL and specifically target tumor cells, several adenoviral-mediated TRAIL gene transfer approaches are currently being exploited using different animal models [19, 20].

However, gene transfer approaches largely depend on the efficient infection of the tumor and avoidance of immune clearance to be effective [21]. In addition, several safety issues concerning the systemic injection of adenoviral vectors still remain to be addressed [22]. For instance, adenoviral gene transfer of TRAIL overcomes an impaired apoptotic response of hepatoma cells, but causes severe apoptosis in primary human hepatocytes [19]. Currently, adenoviral-based gene therapy approaches mainly rely on the intratumoral delivery of TRAIL-encoding adenovirus [23]. Despite a local antitumoral activity, intratumoral delivery of TRAIL has no systemic antitumor activity, thus lacking any clinical value.

It is known that various cell types home preferentially to tumors [24] and can be loaded with the constructs required to produce anticancer molecules. Due to their homing characteristics, CD34+ cells [25] and natural killer (NK) cells [26] may be used as delivery vehicles for anticancer molecules.

Following intravenous infusion, CD34+ cells display specific homing properties, including the capacity to permanently colonize the bone marrow, and transiently colonize the liver and spleen [27-30]. These homing properties are strictly related to the expression of adhesion receptors (e.g., CXCR-4, VLA-4, VLA-5, CD44, etc.) which interact with specific ligands (e.g., SDF-1, VCAM-1, etc.) which are expressed on stromal cells residing within the bone marrow microenvironment as well as the tumor microenvironment [31-34].

NK cells are a subset of lymphocytes that play an essential role in the cellular-based immune defense against tumor cells through major histocompatibility complex non-restricted mechanisms [35]. Following intravenous infusion, NK cells home to the bone marrow and lymphoid organs and extravasate at tumor sites under the influence of appropriate cytokine signals. Many groups have, therefore, sought to use NK cells to home to tumors for therapeutic purposes [36-38].

It has now been found that CD34+ cells and NK cells engineered to express TRAIL can be advantageously exploited for achieving a cell-mediated, anti-tumor activity in vivo.

### DESCRIPTION OF THE INVENTION

According to the present invention, tumor-homing cells are engineered to produce tumor necrosis factor-related apoptosis-inducing ligand (TRAIL) by adenoviral-mediated gene transfer. The cells according to the present invention can be systemically administered to deliver TRAIL to the tumor site without causing the aforementioned drawbacks.

Accordingly, the present invention relates to tumor-homing cells expressing TRAIL and cell preparations containing them. The invention also relates to the use of said cells for the preparation of cell compositions for anti-cancer therapy, in particular for the therapy of human lymphoma.

The cells of the invention can be obtained by transducing tumor-homing cells with a replication-deficient adenovirus encoding the human TRAIL gene (Ad-TRAIL) under the control of a suitable promoter, e.g. the CMV promoter. The transduction can be carried out according to methods well-known to molecular biologists, preferably following the procedure disclosed in the

### examples.

The term "tumor-homing" cells is used according to the invention to define cells able: (1) to home to tumor tissue following intravenous injection, and (2) to express adequate amounts of membrane-bound TRAIL (mTRAIL) for at least a few days.

Examples of tumor-homing cells capable of homing from blood to tumor deposits includes hematopoyetic cells, namely CD34+ hematopoietic cells from the peripheral blood of growth-factor treated cancer patients; NK cells from the peripheral blood; cytokine-induced killer cells (CIK) obtained through ex vivo culture of peripheral blood mononuclear cells; endothelial cells and endothelial progenitor cells; tumor-infiltrating lymphocytes (TIL); lymphokine-activated killer cells (LAK); macrophages; mesenchimal stem cells (MSC) and dendritic cells (DC) (both freshly isolated from peripheral blood or human tissues, or ex vivo expanded), and even human cell lines retaining tumor seeking ability and engineered to express mTRAIL. Several methods can be envisaged in order to obtain adequate expression of mTRAIL, including use of plasmids and viral vectors with appropriate regulatory genetic elements such as tissue-specific promoters and/or enhancers.

CD34+ and NK cells are particularly preferred.

An optimal transduction efficiency of NK cells may be obtained by pre-incubating NK cells for 18 hours with N-acetylcysteine (10 mM). After pre-incubation, NK cells are exposed to graded (50 to 500) Multiplicity of Infection (MOI) of Ad-TRAIL (50 to 500) under serum-free conditions at 37°C. Serum-supplemented medium (RPMI-1640/FBS 20%) is then added, and a few hours later cultures were supplemented with Gene Booster (1:200) and further incubated for 18 hours.

Similar conditions may be used for CD34+ cells, except for the pre-incubation with N-acetylcysteine.

The compositions of the invention can be prepared by using vehicles suitable for parenteral, particularly intravenous, administration, such as those reported in REMINGTON'S PHARMACEUTICAL SCIENCES (Mack Pub. Co., N.J. 1991).

For instance, excipients which may be used include any pharmaceutical agent that is not harmful to the individual receiving the composition, e.g. water, saline, glycerol and ethanol optionally in admixture with auxiliary substances, such as wetting or emulsifying agents, buffers, and the like in. Appropriate doses will depend, among other factors, on the sex, age and conditions of the subject to be treated, the severity of the disease. An appropriate effective amount can be readily determined by any skilled practitioner and may anyhow be determined by means of clinical studies. A therapeutically effective dose will generally be from about 10³ to about 10¹⁵ of transduced cells. Other dosages can be of course established by means of conventional experiments, i.e. relying on dose-response curves, determining the maximal tolerable dose (MTD) or carrying out in a limited number of patients phase I clinical studies. Dosage treatment may be a single dose or a multiple dose schedule. Moreover, the subject may be administered as many doses as appropriate. The skilled practitioner will easily determine the most effective dosage regimen.

The invention will be now illustrated in greater detail by means of the following examples, providing clear and convincing evidence that:
(i) CD34+ stem cells as well as NK cells can be efficiently transduced in vitro with an adenovector expressing TRAIL;
(ii) upon transduction, these cell subtypes transiently express TRAIL on their cell surface;
(iii) such a short duration of TRAIL expression is associated with a potent apoptotic effect detected on both TRAIL-sensitive and TRAIL-resistant tumor cells co-cultured with transduced cells;
(iv) in vivo injection of transduced cells in NOD/SCID bearing an early or advanced stage tumor is associated with a significant prolongation of mice survival, suggesting that both adenoviral transduced CD34+ cells and NK cells may efficiently serve as vehicles for systemic TRAIL delivery for therapeutic purposes.

The effectiveness of intravenous administration provides a distinct advantage of the present invention in comparison protocols based to intra-tumor delivery.

While the mechanism of the in vitro antitumor effect of mTRAIL- expressing human cells is most likely apoptosis triggered by direct reaction between mTRAIL and its cognate receptors on tumor cells, we do not know the mechanism of their in vivo antitumor activity. Direct killing of the tumor cells is possibly coupled with indirect growth-inhibitory effects resulting from apoptosis of tumor-embedded non-tumor cells expressing TRAIL receptors (vascular, peri-vascular, interstitial and other cell types with critical roles in tumor development and tumor cell survival). Ongoing experiments are aimed at better understanding the mechanism of the in vivo observed antitumor activity, even though, of course, the invention will not be limited by any hypothesis on the actual mechanism of the observed activities.

### Example 1 - In vitro triggering of apoptosis in lymphoma cell lines co-cultured with CD34+ cells or NK cells expressing TRAIL following adenoviral-mediated gene transfer

The effects of co-culturing lymphoma cell lines with CD34+ cells or NK cells engineered to express TRAIL following adenoviral-mediated gene transfer were evaluated.

In initial in vitro experiments, an adenoviral vector expressing TRAIL (Ad-TRAIL) was used to set up optimal conditions for adenoviral infection of CD34+ cells and NK cells. The time-course of TRAIL expression on CD34+ and NK cell surface was subsequently monitored by using a PE-conjugated anti-TRAIL monoclonal antibody (clone Rik-2). Finally, the capacity of membrane-bound TRAIL to induce apoptosis of lymphoma cell lines was evaluated by co-culturing Ad-TRAIL/CD34+ cells or Ad-TRAIL/NK cells and lymphoma cell lines.

### Methods

**Adenovirus encoding the human TRAIL gene.** A replication-deficient adenovirus encoding the human TRAIL gene (Ad-TRAIL) expressed from the CMV promoter was used for these experiments [39]. The Ad-TRAIL contains the entire coding sequence of human TRAIL cloned into the *Xho*I and *Not*I sites of pAd5CMVK-NpA. The resultant plasmid and adenovirus backbone sequences (Ad5) that had the E1 genes deleted are transfected into human embryonic kidney 293 cells, and viral particles are isolated and amplified for analysis of TRAIL expression. Recombinant adenoviruses are screened for replication competent virus by A549 plaque assay, and virus titer is determined by plaque assay on 293 cells. Purified viruses are stored in PBS with 3% sucrose and kept at -80°C until use. TRAIL gene product is expressed on the cell surface of transduced cells and can be detected by means of flow cytometry.

**Adenoviral transduction of CD34+ cells**. CD34+ cells to be transduced with Ad-TRAIL were obtained from the peripheral blood of consenting cancer patients receiving chemotherapy and treated with hematopoietic growth factors. CD34+ cells were enriched from leukapheretic samples by means of an immunomagnetic technique and positive selection (Miltenyi Biotech). For adenoviral transduction, CD34+ cells were plated at 2 x 10⁶/ml in 35 mm Petri dishes in 1 ml serum-free medium (IMDM) containing an appropriate dilution of Ad-TRAIL stocks allowing a final multiplicity of infections (MOI) ranging from 100 to 1,000. After incubation (37°C, 5% CO₂) for 2 hours, cultures were supplemented with 1 ml IMDM/FBS20%, and 4 hours later GeneBooster (1:200) was added. Cells were further incubated for 18 hours, then extensively washed (3x) in serum containing medium, and finally transduction efficiency was evaluated by direct immunofluorescence using a PE-conjugated anti-TRAIL antibody.

**Adenoviral transduction of NK cells**. NK cells to be transduced with Ad-TRAIL were obtained from the peripheral blood of consenting healthy donors. NK cells were enriched by means of an immunomagnetic technique and positive selection (Miltenyi Biotech). Before transduction, NK cells were incubated (18 hrs, 37°C) in RPMI-1640 supplemented with 10% FBS and N-acetylcysteine (10 mM). For adenoviral transduction, NK cells were plated at 2 x 10⁶/ml in 35 mm Petri dishes in 1 ml serum-free medium (RPMI-1640) containing an appropriate dilution of Ad-TRAIL stocks allowing a final multiplicity of infections (MOI) ranging from 50 to 500. After incubation (37°C, 5% CO2) for 2 hours, cultures were supplemented with 1 ml RPMI-1640/FBS20%, and 4 hours later GeneBooster (1:200) was added. Cells were further incubated for 18 hours, then extensively washed (3x) in serum containing medium, and finally transduction efficiency was evaluated by direct immunofluorescence using a PE-conjugated anti-TRAIL antibody.

**Co-cultures.** The apoptosis triggering activity of TRAIL expressed on the membrane of CD34+ cells or NK cells was tested in vitro by means of co-culture experiments. Briefly, Ad-TRAIL/CD34+ cells or Ad-TRAIL/NK cells (effector cells) were co-cultured with lymphoma cells (target cells). Apoptosis induction was evaluated 24 and 48 hours after the onset of co-culture. In these experiments, the effector: target cell ratio was calculated on the basis of the transduction efficiency of effector cells.

### Results

**Adenoviral transduction of CD34+ cells**. Preliminary experiments demonstrated that an optimal transduction efficiency of CD34+ cells was consistently achieved by exposing CD34+ cells (2 x 10⁶/ml) to graded MOI of Ad-TRAIL (range, 100 to 1,000) under serum-free conditions for 2 hours (37°C). An equal volume of serum-supplemented medium (IMDM/FBS 20%) was then added, and 4 hours later cultures were supplemented with Gene Booster (1:200) and further incubated for 18 hours. Finally, the cells were extensively washed (3x) in serum containing medium and transgene expression was evaluated by direct immunofluorescence using a PE-conjugated anti-TRAIL antibody.

While no background TRAIL signal was detected in control cells, Ad-exposed cells revealed a percentage of TRAIL-positive CD34+ cells which was increased in an MOI-dependent manner. In a representative experiment, the percentages of TRAIL-positive CD34+ cells were 25% and 84% at an MOI of 100 and 1000, respectively.

The above described infection protocol consistently resulted in a maximal gene transfer efficiency at an MOI of 1,000 with a mean (±SD) of 83 ± 8% (range 70 - 95%, n = 5) CD34+ cells expressing TRAIL. Cell viability, as evaluated by the Trypan blue dye exclusion test, was unaffected by an MOI as high as 1,000 (with ≥85% viable cells).

To evaluate the time-course of transgene expression, CD34+ cells were transduced with an MOI of 1,000 and analyzed for TRAIL expression up to 7 days after transduction. In these experiments, in order to allow CD34+ cell survival, cultures were supplemented with low dose (3 ng/ml) of granulocyte colony-stimulating factor (G-CSF). As shown in table 1, a substantial percentage of CD34+ cells (25%) continued to express TRAIL up to 120 hours following transduction.

**Table 1 - Overtime transgene expression of CD34+ cells transduced with Ad-TRAIL**

| | | | | | |
|---|---|---|---|---|---|
| **Hours post-infection** | 24 | 48 | 72 | 120 | 168 |
| **% of CD34* cells expressing TRAIL** | 95 | 70 | 42 | 25 | 13 |

**Adenoviral transduction of NK cells.** Preliminary experiments demonstrated that an optimal transduction efficiency of NK cells was consistently achieved by pre-incubating NK cells for 18 hours with N-acetylcysteine (10 mM). After pre-incubation, NK cells (2 x 10⁶/ml) were exposed to graded MOI of Ad-TRAIL (range, 50 to 500) under serum-free conditions for 2 hours (37°C). An equal volume of serum-supplemented medium (RPMI-1640/FBS 20%) was then added, and 4 hours later cultures were supplemented with Gene Booster (1:200) and further incubated for 18 hours. Finally, the cells were extensively washed (3x) in serum containing medium and transgene expression was evaluated by direct immunofluorescence using a PE-conjugated anti-TRAIL antibody.

Ad-exposed cells revealed a percentage of TRAIL-positive NK cells which was increased in an MOI-dependent manner. In a representative experiment, the percentages of TRAIL-positive NK cells were 30%, 45% and 46% at an MOI of 50, 100 and 500, respectively.

The above described infection protocol consistently resulted in a maximal gene transfer efficiency at an MOI of 100 with a mean (±SD) of 61 ± 18% (range 45 - 84%, n = 4) NK cells expressing TRAIL. Cell viability, as evaluated by the Trypan blue dye exclusion test, was unaffected by an MOI as high as 100 (with ≥85% viable cells).

To evaluate the time-course of transgene expression, NK cells were transduced with an MOI of 100 and analyzed for TRAIL expression up to 7 days after transduction. As shown in table 2, transduced NK cells continued to express TRAIL up to 168 hours following transduction.

**Table 2 - Overtime transgene expression of NK cells transduced with Ad-TRAIL**

| | | | | | |
|---|---|---|---|---|---|
| **Hours post-infection** | 24 | 48 | 72 | 120 | 168 |
| **% of CD34* cells expressing TRAIL** | 41 | 55 | 60 | 63 | 55 |

**Co-cultures.** Finally, we evaluated the capacity of membrane-bound TRAIL to trigger apoptosis in lymphoid cell lines by co-culturing Ad-TRAIL/CD34+ cells or Ad-TRAIL/NK cells (effector cells) and tumor cell lines (target cells). For these experiments, two cell lines were selected according to their sensitivity to soluble TRAIL. In particular, the TRAIL-sensitive KMS-11 cell line and the TRAIL-resistant JVM-2 cell line were used.

CD34+ cells or NK cells transduced under optimal conditions according to the infection protocols described above were collected 24 hours after the initial exposure to adenovirus, extensively washed and co-cultured with KMS-11 or JVM-2 cells.

When Ad-TRAIL/CD34+ cells were co-cultured at an effector: target cell ratio of 0.8:1, a substantial proportion (81%) of apoptotic and necrotic cells was detected for KMS-11 cells after a 24-hour co-culture. The amount of apoptotic cells was further increased after a 48-hour co-culture (93% apoptotic cells).

When Ad-TRAIL/NK cells were co-cultured at an effector: target cell ratio of 0.6:1, a substantial proportion (83%) of apoptotic and necrotic cells was detected for KMS-11 cells after a 24-hour co-culture. The amount of apoptotic cells was further increased after a 48-hour co-culture (85% apoptotic cells).

For the TRAIL-sensitive KMS-11 cell line, the potency of the apoptotic effect exerted by membrane-bound TRAIL was similar to that exerted by a 24- to 48-hour exposure to a high-dose (100 ng/ml) of soluble TRAIL (Table 3). However, given the pharmacokinetic profile of TRAIL after intravenous injection (plasmatic half-life of 32 minutes and elimination half-life of 4.8 hours), a continuous 48- or even 24-hour exposure at 100 ng/ml cannot be achieved in vivo.

**Table 3 - Effect of soluble TRAIL on KMS-11 cells**

| **Amount of soluble TRAIL** | **% of viable cells after 24 hours** | **% of viable cells after 48 hours** |
|---|---|---|
| 0 ng/ml | 78 | 75 |
| 100 ng/ml | 27 | 2 |

Co-culture experiments were also performed by incubating Ad-TRAIL/CD34+ cells or Ad-TRAIL/NK cells with the soluble TRAIL-resistant JVM-2 cell line.

When Ad-TRAIL/CD34+ cells were co-cultured at an effector: target cell ratio of 0.8:1, a substantial proportion (51%) of apoptotic and necrotic cells was detected for the TRAIL-resistant JVM-2 cells after a 48-hour co-culture.

When Ad-TRAIL/NK cells were co-cultured at an effector: target cell ratio of 0.6:1, a substantial proportion (53%) of apoptotic and necrotic cells was detected for the TRAIL-resistant JVM-2 cells after a 48-hour co-culture.

For the TRAIL-resistant JVM-2 cell line, the potency of the apoptotic effect exerted by membrane-bound TRAIL was significantly higher than that exerted by a 48-hour exposure to a high-dose (100 ng/ml) of soluble TRAIL (table 4).

**Table 4 - Effect of soluble TRAIL on JVM-2 cells**

| **Amount of soluble TRAIL** | **% of viable cells after 24 hours** | **% of viable cells after 48 hours** |
|---|---|---|
| 0 ng/ml | 87 | 84 |
| 100 ng/ml | 86 | 80 |

To further explore the potency of the apoptotic activity of cell-mediated TRAIL delivery, effector cells (Ad-TRAIL/CD34+ cells or Ad-TRAIL/NK cells) were co-cultured with target cells (KMS-11 cell line) for 24 hours at different effector: target ratios.

As shown in table 5, Ad-TRAIL/CD34+ cells triggered a substantial percentage (66%) of KMS-11 cells to undergo apoptosis or necrosis in co-cultures set-up with an effector: target ratio of 0.4:1.

**Table 5 - Induction of apoptosis by co-culturing Ad-TRAIL/CD34+ cells and KMS-11 cells for 24 hours at increasing E:T ratios**

| | | | | |
|---|---|---|---|---|
| **CD34+/KMS-11 ratio** | 0:1 | 0.08:1 | 0.4:1 | 0.8:1 |
| **% of residual viable cells** | 71 | 67 | 34 | 22 |

As shown in table 6, Ad-TRAIL/NK cells triggered a substantial percentage (64%) of KMS-11 cells to undergo apoptosis or necrosis in co-cultures set-up with an effector: target ratio of 0.3:1.

**Table 6 - Induction of apoptosis by co-culturing Ad-TRAIL/NK cells and KMS-11 cells for 24 hours at increasing E:T ratios**

| | | | | |
|---|---|---|---|---|
| **NK/KMS ratio** | 0:1 | 0.06:1 | 0.3:1 | 0.6:1 |
| **% of residual viable cells** | 66 | 68 | 36 | 19 |

### Example 2 - In vivo evaluation in NOD/SCID mice of the anticancer activity of CD34+ cells or NK cells engineered to express TRAIL following adenoviral-mediated gene transfer

In order to investigate the therapeutic potential of Ad-TRAIL/CD34+ cells or Ad-TRAIL/NK cells, NOD/SCID mice were reinfused with the TRAIL-sensitive KMS-11 multiple myeloma cell line. Subsequently, mice were injected with Ad-TRAIL/CD34+ cells or Ad-TRAIL/NK cells and mice survival was used as a readout of the antitumor efficacy of cell-based TRAIL delivery.

### Methods

**Evaluation of the antitumor activity of Ad-TRAIL/CD34+ cells or Ad-TRAIL/NK cells.** Six- to eight-wk-old female NOD/SCID mice with body weight of 20 to 25 g, were purchased from Charles River (Milano, Italy). Mice were housed under standard laboratory conditions according to our institutional guidelines. Experimental procedures performed on animals were approved by the Ethical Committee for Animal Experimentation of the Istituto Nazionale Tumori and were carried out in accordance with the guidelines of the United Kingdom Coordinating Committee on Cancer Research.

Mice were inoculated intravenously (IV) with KMS-11 cells (0.5 x 10⁶ per mouse). Treatment with Ad-TRAIL/CD34+ cells or Ad-TRAIL/NK cells (1 x 10⁶ per mouse) consisted in IV weekly injections for 4 weeks starting either on day 7 or 17 after tumor cell inoculation. The mean transduction efficiencies of reinfused Ad-TRAIL/CD34+ cells or Ad-TRAIL/NK cells were 83 ± 8% and 61 ± 18%, respectively. Thus, each mouse received an average number of 3.32 x 10⁶ TRAIL-expressing CD34+ cells and 2.44 x 10⁶ TRAIL-expressing NK cells over four injections. Mice were checked twice weekly for tumor appearance, body weight measurements and toxicity. The survival times of animals in each group were recorded and differences among the groups were evaluated by statistical analysis. Appropriate control groups included: (i) mice injected with tumor cells only, (iii) mice injected with tumor cells plus non-transduced CD34+ cells or NK cells.

### Results

**Ad-TRAIL/CD34+ cells.** Mice xenografted with KMS-11 cells (0.5 x 10⁶ per mouse) were treated with 4 IV injections of Ad-TRAIL/CD34+ cells on a weekly basis according to two different schedules, i.e., treatment of early stage tumor which was started on day 7, and treatment of advanced stage tumor which was started on day 17 after KMS-11 cell injection. Since the mean transduction efficiency of reinfused Ad-TRAIL/CD34+ cells was 83 ± 8% (mean ± SD, n = 9), each mouse received an average number of 3.32 x 10⁶ TRAIL-expressing CD34+ cells over four injections.

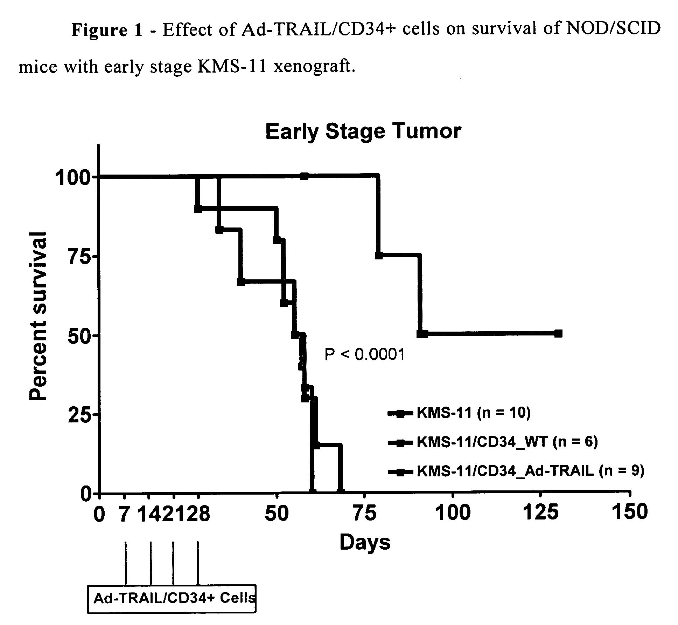

As shown in Figure 1, the median survival of NOD/SCID mice injected with KMS-11 cells alone was 56 days. Injection of wild type CD34+ cells had no effect on median survival (56 days), whereas NOD/SCID mice treated with Ad-TRAIL/CD34+ cells for an early stage tumor showed a median survival of 111 days (P ≤ 0.0001).

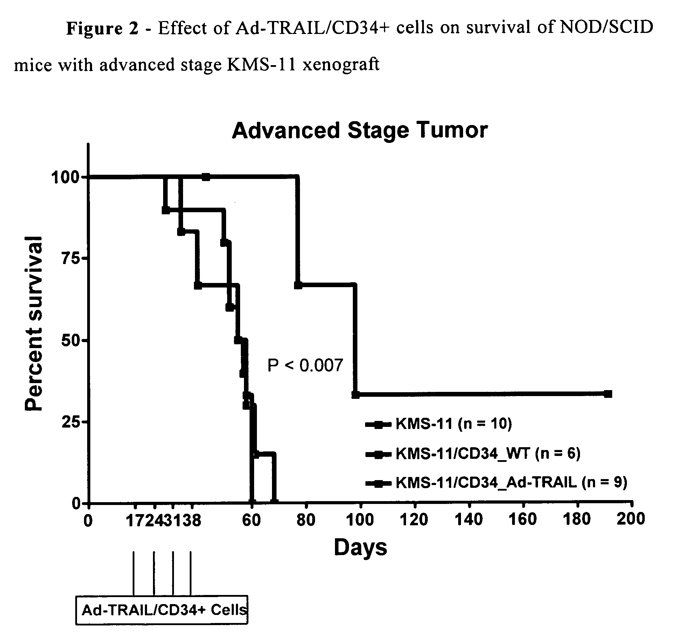

The effect of treating an advanced stage tumor with Ad-TRAIL/CD34+ cells is depicted in Figure 2. The median survival of NOD/SCID mice injected with KMS-11 cells alone was 56 days. Injection of wild type CD34+ cells had no effect on median survival (56 days), whereas treatment with Ad-TRAIL/CD34+ cells of advanced stage tumor was associated with a significant increase of median survival (98 days, P ≤ 0.007).

Ad-TRAIL/NK cells. Mice xenografted with KMS-11 cells (0.5 x 10⁶ per mouse) were treated with 4 IV injections of Ad-TRAIL/NK cells on a weekly basis. Reinfusions of NK cells were started on day 7 after KMS-11 cell injection, at an early stage of tumor growth. For this experiment, the mean transduction efficiency of reinfused Ad-TRAIL/NK cells was 61 ± 18%. Thus, each mouse received an average number of 2.44 x 10⁶ TRAIL-expressing NK cells over four injections.

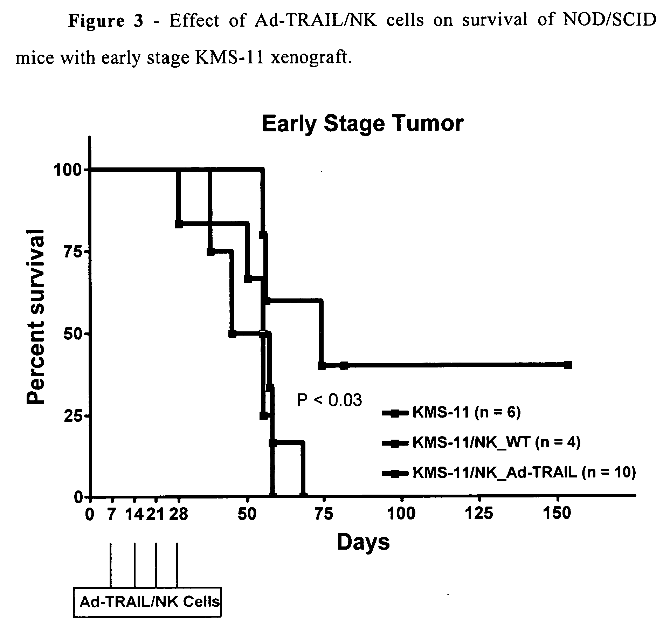

As shown in Figure 3, the median survival of NOD/SCID mice injected with KMS-11 cells alone was 56 days. Injection of wild type NK cells had no effect on median survival (50 days), whereas treatment with Ad-TRAIL/NK cells of early stage tumor was associated with a significant increase of the median survival (74 days, P ≤ 0.03).

### REFERENCES

1. Danial, N.N. and S.J. Korsmeyer, *Cell death: critical control points.* Cell, 2004. **116**(2): p. 205-19.
2. Blagosklonny, M.V., *Prospective strategies to enforce selectively cell death in cancer cells.* Oncogene, 2004. **23**(16): p. 2967-75.
3. Waxman, D.J. and P.S. Schwartz, *Harnessing apoptosis for improved anticancer gene therapy.* Cancer Res, 2003. **63**(24): p. 8563-72.
4. Ashkenazi, A. and V.M. Dixit, *Death receptors: signaling and modulation.* Science, 1998. **281**(5381): p. 1305-8.
5. Ashkenazi, A. and V.M. Dixit, *Apoptosis control by death and decoy receptors.* Curr Opin Cell Biol, 1999. **11**(2): p. 255-60.
6. Wang, S. and W.S. El-Deiry, *TRAIL and apoptosis induction by TNF-family death receptors.* Oncogene, 2003. **22**(53): p. 8628-33.
7. Almasan, A. and A. Ashkenazi, *Apo2L*/*TRAIL: apoptosis signaling, biology, and potential for cancer therapy.* Cytokine Growth Factor Rev, 2003. **14**(3-4): p. 337-48.
8. LeBlanc, H., et al., *Tumor-cell resistance to death receptor-induced apoptosis through mutational inactivation of the proapoptotic Bcl-2 homolog Bax.* Nat Med, 2002. **8**(3): p. 274-81.
9. Ashkenazi, A., et al., *Safety and antitumor activity of recombinant soluble Apo2 ligand.* J. Clin. Invest., 1999. **104**(2): p. 155-162.
10. Walczak, H., et al., *Tumoricidal activity of tumor necrosis factor-related apoptosis-inducing ligand in vivo.* Nat Med, 1999. **5**(2): p. 157-63.
11. Mitsiades, C.S., et al., *TRAIL*/*Apo2L ligand selectively induces apoptosis and overcomes drug resistance in multiple myeloma: therapeutic applications.* Blood, 2001. **98**(3): p. 795-804.
12. Fulda, S., et al., *Smac agonists sensitize for Apo2L*/*TRAIL- or anticancer drug-induced apoptosis and induce regression of malignant glioma in vivo.* Nature Medicine, 2002. **8**(8): p. 808-815.
13. Pollack, I.F., M. Erff, and A. Ashkenazi, *Direct stimulation of apoptotic signaling by soluble Apo2L*/*tumor necrosis factor-related apoptosis-inducing ligand leads to selective killing of glioma cells.* Clinical Cancer Research, 2001. **7**(5): p. 1362-1369.
14. Lawrence, D., et al., *Differential hepatocyte toxicity of recombinant Apo2L*/*TRAIL versions.* Nat Med, 2001. **7**(4): p. 383-5.
15. Qin, J., et al., *Avoiding premature apoptosis of normal epidermal cells.* Nature Medicine, 2001. **7**(4): p. 385-386.
16. Jo, M., et al., *Apoptosis induced in normal human hepatocytes by tumor necrosis factor-related apoptosis-inducing ligand.* Nat Med, 2000. **6**(5): p. 564-7.
17. Johnston, J.B., et al., *Role of the TRAIL*/*APO2-L death receptors in chlorambucil- and fludarabine-induced apoptosis in chronic lymphocytic leukemia.* Oncogene, 2003. **22**(51): p. 8356-69.
18. Yamanaka, T., et al., *Chemotherapeutic agents augment TRAIL-induced apoptosis in human hepatocellular carcinoma cell lines.* Hepatology, 2000. **32**(3): p. 482-90.
19. Armeanu, S., et al., *Adenoviral gene transfer of tumor necrosis factor-related apoptosis-inducing ligand overcomes an impaired response of hepatoma cells but causes severe apoptosis in primary human hepatocytes.* Cancer Res, 2003. **63**(10): p. 2369-72.
20. Voelkel-Johnson, C., D.L. King, and J.S. Norris, *Resistance of prostate cancer cells to soluble TNF-related apoptosis-inducing ligand (TRAIL*/*Apo2L) can be overcome by doxorubicin or adenoviral delivery of full-length TRAIL.* Cancer Gene Ther, 2002. **9**(2): p. 164-72.
21. McCormick, F., *Cancer gene therapy: fringe or cutting edge?* Nat Rev Cancer, 2001. **1**(2): p. 130-41.
22. Somia, N. and I.M. Verma, *Gene therapy: trials and tribulations.* Nat Rev Genet, 2000. g(2): p. 91-9.
23. Griffith, T.S. and E.L. Broghammer, *Suppression of tumor growth following intralesional therapy with TRAIL recombinant adenovirus*. Mol Ther, 2001. **4**(3): p. 257-66.
24. Harrington, K., et al., *Cells as vehicles for cancer gene therapy: the missing link between targeted vectors and systemic delivery?* Hum Gene Ther, 2002. **13**(11): p. 1263-80.
25. Rafii, S. and D. Lyden, *Therapeutic stem and progenitor cell transplantation for organ vascularization and regeneration.* Nat Med, 2003. **9**(6): p. 702-12.
26. Rosenberg, S.A., *Karnofsky Memorial Lecture. The immunotherapy and gene therapy of cancer*. J Clin Oncol, 1992. **10**(2): p. 180-99.
27. Hardy, C.L., *The homing of hematopoietic stem cells to the bone marrow.* Am J Med Sci, 1995. **309**(5): p. 260-6.
28. Lapidot, T., *Mechanism of human stem cell migration and repopulation of NOD*/*SCID and B2mnull NOD*/*SCID mice. The role of SDF-1*/*CXCR4 interactions.* Ann N Y Acad Sci, 2001. **938**: p. 83-95.
29. Lapidot, T. and I. Petit, *Current understanding of stem cell mobilization: the roles of chemokines, proteolytic enzymes, adhesion molecules, cytokines, and stromal cells.* Exp Hematol, 2002. **30**(9): p. 973-81.
30. Srour, E.F., et al., *Homing, cell cycle kinetics and fate of transplanted hematopoietic stem cells. Leukemia*, 2001. **15**(11): p. 1681-4.
31. Clark, B.R., J.T. Gallagher, and T.M. Dexter, *Cell adhesion in the stromal regulation of haemopoiesis.* Baillieres Clin Haematol, 1992. **5**(3): p. 619-52.
32. Deguchi, T. and Y. Komada, *Homing-associated cell adhesion molecule (H-CAM*/*CD44) on human CD34+ hematopoietic progenitor cells.* Leuk Lymphoma, 2000. **40**(1-2): p. 25-37.
33. Tavassoli, M. and J.J. *Minguell, Homing of hemopoietic progenitor cells to the marrow.* Proc Soc Exp Biol Med, 1991. **196**(4): p. 367-73.
34. Verfaillie, C.M., *Adhesion Receptors as Regulators of the Hematopoietic Process.* Blood, 1998. **92**(8): p. 2609-2612.
35. Cooper, M.A., T.A. Fehniger, and M.A. Caligiuri, *The biology of human natural killer-cell subsets.* Trends Immunol, 2001. **22**(11): p. 633-40.
36. Basse, P.H., T.L. Whiteside, and R.B. Herberman, *Use of activated natural killer cells for tumor immunotherapy in mouse and human.* Methods Mol Biol, 2000. **121**: p. 81-94.
37. deMagalhaes-Silverman, M., et al., *Posttransplant adoptive immunotherapy with activated natural killer cells in patients with metastatic breast cancer.* J Immunother, 2000. **23**(1): p. 154-60.
38. Rosenberg, S.A., et al., *Prospective randomized trial of high-dose interleukin-2 alone or in conjunction with lymphokine-activated killer cells for the treatment of patients with advanced cancer.* J Natl Cancer Inst, 1993. **85**(8): p. 622-32.
39. Griffith, T.S., et al., *Adenoviral-mediated transfer of the TNF-related apoptosis-inducing ligand*/*Apo-2 ligand gene induces tumor cell apoptosis*. J Immunol, 2000. **165**(5): p. 2886-94.

## Claims

1. Tumor-homing cells expressing the tumor necrosis factor-related apoptosis inducing ligand.

2. Cells according to claim 1, obtainable by transducing tumor-homing cells with adenoviral vectors coding for the tumor necrosis factor-related apoptosis inducing ligand.

3. Cells according to claim 1 or 2 wherein said tumor-homing cells are selected from CD34+ hematopoietic cells from the peripheral blood of growth-factor treated cancer patients; NK cells from the peripheral blood; cytokine-induced killer cells (CIK) obtained through ex vivo culture of peripheral blood mononuclear cells;endothelial cells and endothelial progenitor cells; tumor-infiltrating lymphocytes (TIL);lymphokine-activated killer cells (LAK); macrophages; mesenchimal stem cells (MSC) and dendritic cells (DC).

4. Cells according to claim 3, obtainable by transducing NK or CD34+ cells with adenoviral vectors coding for the tumor necrosis factor-related apoptosis inducing ligand.

5. Cell preparations containing the cells of claims 1-4 in admixture with physiologically acceptable vehicles.

6. Use of the cells of claims 1-4 for the preparation of a medicament for the treatment of tumors.

7. Use according to claim 6, wherein the tumor is a lymphoma.

8. Use according to claim 6 or 7, wherein the medicament is administered intravenously.
